# EUROPEAN PATENT APPLICATION

(11) **EP 4 074 360 A1**
(43) Date of publication of application: **19.10.2022**
(21) Application number: 22158706.6
(22) Date of filing: 25.02.2022
(51) Int. Cl.: A61M 16/10, A61M 16/12, A61M 16/00, A61M 16/20

(54) **A METHOD OF CONTROLLING A HIGH FLOW OXYGEN DEVICE**

(30) Priority: 12.04.2021 TR 202106458
(71) Applicant: Arçelik Anonim Sirketi, 34445 Istanbul (TR)
(72) Inventor: DINCER, Mehmet Onur, 34445 ISTANBUL (TR); KANDEMIR, Nihat, 34445 ISTANBUL (TR); KERPICCI, Husnu, 34445 ISTANBUL (TR)

(57) **Abstract**

The present invention relates to a method of controlling the operation of a high flow oxygen device (1) comprising the steps of; providing a gas flow block (2) having a first inlet and a second inlet for introducing oxygen and ambient air into the gas flow block (2) respectively, providing a blower (3) for introducing ambient air into the gas flow block (2) via the second inlet, providing a first valve (4) and a second valve (5) on the gas flow block (2) for regulating the flow of oxygen and ambient air respectively, providing a first flow sensor (6) in-between the gas flow block (2) and a gas mixture block (7) to measure the pressure of the oxygen, and providing a bidirectional flow sensor (8) in-between the gas flow block (2) and the gas mixture block (7) to measure the pressure of the ambient air, wherein the pressure is positive if the ambient air is flowing from the gas flow block (2) towards the gas mixture block (7) and wherein the pressure is negative if the ambient air is flowing from the gas mixture block (7) towards the gas flow block (2), mixing the oxygen and the ambient air in the gas mixture block (7) and transmitting the mixture to an expiratory circuit via an outlet (9), providing a control unit to control the said valves (4,5), sensors (6,8) and the blower (3) such that the oxygen percentage of the mixture at the outlet (9) can be adjusted.

## Description

The present invention relates to a high flow oxygen device, in particular to a method of controlling a high flow oxygen device.

High flow oxygen devices are used for supporting patients having difficulty in breathing. High flow oxygen devices generally use an inlet for oxygen and another inlet for introducing ambient air into a mixing chamber. After mixing oxygen and ambient air, the mixture is transmitted to a patient. In some cases, the patient may require %90 or a higher percentage of oxygen to sustain himself. If the required gas is mainly oxygen, the ambient air is not sucked into the high flow oxygen device. In some high flow oxygen devices, having a flow path, some of this oxygen may flow in a reverse direction, following the path of the incoming ambient air. Afterwards, oxygen enters the casing of the high flow oxygen devices, resulting in a loss of pressure and possible fire hazards.

A prior art publication in the technical field of the present invention may be referred to as US2017002830A1 among others, the document disclosing a blower for a breathing apparatus having a diffuser for increasing static pressure and/or reducing noise and/or mitigating pressure instabilities and/or managing reverse flow.

A prior art publication in the technical field of the present invention may be referred to as US2019307978A1 among others, wherein the document discloses control methods that can indirectly determine incorrect connections between components in a respiratory therapy system.

A prior art publication in the technical field of the present invention may be referred to as WO2020204731A1 among others, wherein various control methods can indirectly determine incorrect connections between components in a respiratory therapy system.

An objective of the present invention is to provide a high flow oxygen device having improved safety.

The method realized to achieve the aim of the present invention and disclosed in the first claim and the dependent claims comprises a high flow oxygen device. A gas flow block having a first and a second inlet are provided on the gas flow block. The first inlet is provided for introducing oxygen into the gas flow block from an external oxygen source such as a pressurized oxygen tank, or an oxygen main of a hospital. The second inlet is used for introducing ambient air into the gas flow block by means of a blower. The blower can be a fan or an air pump etc. Ambient air and oxygen are not mixed inside the gas flow block but flows through separate paths. The gas flow block is further provided with a first valve and a second valve. The said valves are used to regulate the flow of oxygen and ambient air respectively. The first and second valves volumetrically control the flow of oxygen and ambient air. The gas flow block is gaseously connected to a gas mixture block wherein oxygen and ambient air is mixed. This connection is achieved by means of a pair of pipes. The pipes comprise a first flow sensor and a bidirectional flow sensor. The first flow sensor measures the pressure of oxygen passing through whereas the bidirectional flow sensor measures the pressure of ambient air passing through. The bidirectional flow sensor measures the pressure in both directions. Meaning that, when and if ambient air flows from the gas flow block towards the gas mixture block, the pressure reading is positive, whereas when and if ambient air flows from the gas mixture block towards the gas flow block, the pressure reading is negative. Likewise, the first flow sensor measures the pressure of oxygen passing through it. Afterwards, oxygen and ambient air is mixed inside the gas mixture block and is transmitted towards an outlet which is connected to an expiratory circuit used for ventilating a patient. The said valves, the bidirectional flow sensor, the first flow sensor and blower is connected to a control unit. This connection is achieved by electrical wiring but a wireless method can also be used. The control unit, operates the said valves at a required volumetric capacity, thus achieving a predetermined oxygen percentage at the outlet. In case, wherein a high oxygen concentration is required, the control unit can turn off the blower, thus transmitting pure or mostly oxygen towards the outlet. In such cases, and due to the pressure of the oxygen, pure oxygen tends to flow backwards from the gas mixture block and towards the bidirectional flow sensor and finally into the casing of the high flow oxygen device by passing through the blower. In this case, the pressure reading received from bidirectional flow sensor is negative and thusly, the control unit detects the reverse flow. Based on this, the control unit activates the blower, thus forcing a certain amount of ambient air towards the bidirectional flow sensor such that the pressure reading is equal or slightly more than zero. As a result of this, oxygen leakage into the casing is prevented. This is vital because oxygen is highly flammable. High concentration of oxygen inside the casing may ignite due to sparks created by electrical components provided therein. By means of this invention, oxygen leakages and therefore possible fire risks are avoided.

In an embodiment of the invention, the first flow sensor is also a bidirectional sensor.

In an embodiment of the invention, ambient air is introduced into the gas flow block by means of a hose provided between the blower and the second inlet. By means of the hose, the time required for oxygen to flow back into the casing is prolonged, thus improving response time of the control unit in case of negative pressure reading coming from bidirectional flow sensor.

In an embodiment of the invention, the high flow oxygen device comprises a tracer source provided in close vicinity of the first inlet. The tracer source releases a tracer material into the oxygen. The high flow oxygen device further comprises a sensor that is used to detect the tracer. The sensor is provided in close vicinity of the blower and detects the tracer when and if it reaches the inner cavity of the high flow oxygen device and informs the control unit accordingly. The tracer source and the tracer provides a secondary control mechanism for oxygen leakages.

By means of the present invention, oxygen leakages and possibly fires resulting therefrom are prevented.

The drawings are not meant to delimit the scope of protection as identified in the claims nor should they be referred to alone in an effort to interpret the scope identified in the claims without recourse to the technical disclosure in the description of the present invention.

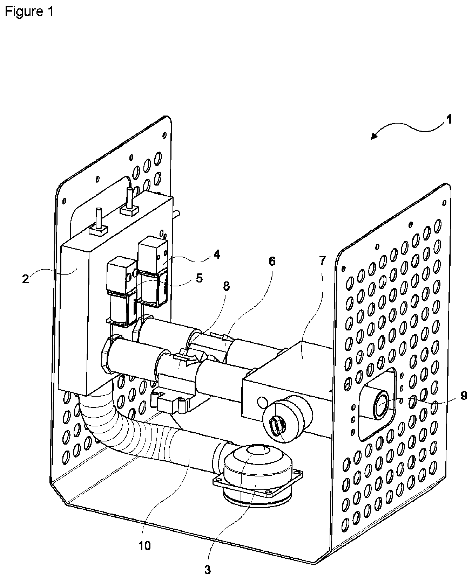

Figure 1 - is a perspective view of the high flow oxygen device

The following numerals are assigned to different parts demonstrated in the drawings and referred to in the present detailed description of the invention:
1. High flow oxygen device
2. Gas flow block
3. Blower
4. First valve
5. Second valve
6. First flow sensor
7. Gas mixture block
8. Bidirectional flow sensor
9. Outlet
10. Hose

The present invention relates to a method of controlling the operation of a high flow oxygen device (1) comprising the steps of; providing a gas flow block (2) having a first inlet and a second inlet for introducing oxygen and ambient air into the gas flow block (2) respectively, providing a blower (3) for introducing ambient air into the gas flow block (2) via the second inlet, providing a first valve (4) and a second valve (5) on the gas flow block (2) for regulating the flow of oxygen and ambient air respectively, providing a first flow sensor (6) in-between the gas flow block (2) and a gas mixture block (7) to measure the pressure of the oxygen, and providing a bidirectional flow sensor (8) in-between the gas flow block (2) and the gas mixture block (7) to measure the pressure of the ambient air, wherein the pressure is positive if the ambient air is flowing from the gas flow block (2) towards the gas mixture block (7) and wherein the pressure is negative if the ambient air is flowing from the gas mixture block (7) towards the gas flow block (2), mixing the oxygen and the ambient air in the gas mixture block (7) and transmitting the mixture to an expiratory circuit via an outlet (9), providing a control unit to control the said valves (4,5), sensors (6,8) and the blower (3) such that the oxygen percentage of the mixture at the outlet (9) can be adjusted.

The present invention further involves the step wherein the control unit activates the blower (3) such that the pressure measured by the bidirectional flow sensor (8) is equal or greater than zero if the required mixture is at least %90 oxygen. The high flow oxygen device (1) comprise the gas flow block (2). The gas flow block (2) comprises the first inlet via which oxygen is introduced into the gas flow block (2) and a second inlet via which ambient air is introduced into the gas flow block (2). Ambient air and oxygen passes through different lines inside the gas flow block (2) and are not mixed with each other. Ambient air is provided by means of a blower (3) provided inside the casing of the high flow oxygen device (1). The first valve (4) is used for regulating the flow of oxygen whereas the second valve (5) is used for regulating the flow of ambient air. In this preferred embodiment, said valves (4,5) are provided on the gas flow block (2), however said valves (4,5) can be provided anywhere inside casing of the high flow oxygen device (1). The high flow oxygen device (1) further comprises the gas mixture block (7) wherein the oxygen and ambient air is mixed. The gas mixture block (7) is in gaseous connection to the gas flow block (2) by means of a pair of pipes. The first flow sensor (6) is provided on one of the said valves and measures the pressure of oxygen passing through the said pipe. On the other hand, a bidirectional flow sensor (8) is provided on the other one of the said pipes and measures the pressure of ambient air passing through the said pipe. By means of the bidirectional flow sensor (8) and the first flow sensor (6), the pressure and therefore the amount of gas passing through the respective pipes are measured accurately. The bidirectional flow sensor (8) allows the measurement of the gas passing in both directions. When the gas is flowing through the gas flow block (2) towards the gas mixture block (7), the pressure reading is a positive value whereas when the gas is flowing through gas mixture block (7) towards the gas flow block (2), the pressure reading is a negative value. As a result of this, the reverse flow can be detected. Afterwards, ambient air and oxygen is mixed inside the gas mixture block (7). The high flow oxygen device (1) further comprises the outlet (9) via which the mixed air is transferred to the expiratory circuit which is connected to a patient to supply the patient with the mixture of ambient air and oxygen or pure oxygen, depending on the respiratory needs of the patient. The high flow oxygen device (1) is further provided with the control unit (not shown), wherein the control unit controls the flow rate through first valve (4) and the second valve (5), measures the amount of pressure read by the first flow sensor (6) and the bidirectional flow sensor (8) and activates or deactivates the blower (3). As a result of this, the oxygen concentration of the mixture exiting the outlet (9) can be adjusted. The high flow oxygen device (1) is also provided with a display wherein the user may adjust the oxygen ratio of the mixture exiting through the outlet (9). Upon entering the requested value, the control unit activates the said valves (4,5) and adjusts the oxygen concentration of the mixture to be transferred to the patient. In case, wherein a high percentage of oxygen concentration is required, the blower (3) is turned off or is run at a lower speed thus achieving a higher percentage of oxygen in the final mixture. If the required oxygen concentration is %90 or higher, the blower (3) is activated at a minimum speed or completely turned off. Likewise, if oxygen concentration of %100 is required, the blower is turned off completely. In such cases, the oxygen flows back into the casing of the high flow oxygen device (1) by flowing through the gas mixture block (7) and the bidirectional flow sensor (8). In this case, the control unit detects a negative pressure reading via the bidirectional flow sensor (8), indicating that oxygen is flowing backwards. The control unit, upon detecting the reverse flow of the oxygen, activates or increases the load on the blower (3). The blower (3) is not activated at full capacity but only enough to get a pressure reading equal or slightly more than zero at bidirectional flow sensor (8). As a result of this, it is ensured that the oxygen can not reach the inner casing of the high flow oxygen device (1), thus eliminating the possibility of oxygen chemically reacting with the outer environment, thereby preventing a possible fire. By means of this invention, fire safety is achieved.

In a further embodiment of the invention, the first flow sensor (6) is a bidirectional flow sensor.

In an embodiment of the invention, the ambient air is introduced into the second inlet by means of a hose (10) between the second inlet and the blower (3). The hose (10) provides a longer path for oxygen to pass before entering the interior of the casing, thus improving safety and response time of the control unit.

In an embodiment of the invention, the high flow oxygen device (1) comprises a tracer source provided in close vicinity of the first inlet, releasing a tracer material into the oxygen, and a sensor in close vicinity of the blower (3), detecting the tracer wherein the control unit activates the blower (3) if the sensor detects tracer material such that the pressure reading of the bidirectional flow sensor (8) becomes equal or greater than zero. The tracer and the tracer source further improves the safety of the high flow oxygen device (1) against fire.

An advantageous effect provided by means of the invention is that the oxygen is prevented to reach the casing of the high flow oxygen device (1), thus preventing the interaction of oxygen with the electrical components of the high flow oxygen device (1) thereby preventing the possibility of a fire.

## Claims

1. A method of controlling the operation of a high flow oxygen device (1) comprising the steps of;
providing a gas flow block (2) having a first inlet and a second inlet for introducing oxygen and ambient air into the gas flow block (2) respectively, providing a blower (3) for introducing ambient air into the gas flow block (2) via the second inlet,
providing a first valve (4) and a second valve (5) on the gas flow block (2) for regulating the flow of oxygen and ambient air respectively,
providing a first flow sensor (6) in-between the gas flow block (2) and a gas mixture block (7) to measure the pressure of the oxygen, and
providing a bidirectional flow sensor (8) in-between the gas flow block (2) and the gas mixture block (7) to measure the pressure of the ambient air, wherein the pressure is positive if the ambient air is flowing from the gas flow block (2) towards the gas mixture block (7) and wherein the pressure is negative if the ambient air is flowing from the gas mixture block (7) towards the gas flow block (2),
mixing the oxygen and the ambient air in the gas mixture block (7) and transmitting the mixture to an expiratory circuit via an outlet (9),
providing a control unit to control the said valves (4,5), sensors (6,8) and the blower (3) such that the oxygen percentage of the mixture at the outlet (9) can be adjusted,
wherein
the control unit activates the blower (3) such that the pressure measured by the bidirectional flow sensor (8) is equal or greater than zero if the required mixture is at least %90 oxygen.

2. Method of controlling the operation of a high flow oxygen device (1) according to claim 1, wherein the first flow sensor (6) is a bidirectional flow sensor.

3. Method of controlling the operation of a high flow oxygen device (1) according to any preceding claim, wherein the ambient air is introduced into the second inlet by means of a hose (10) between the second inlet and the blower (3).

4. Method of controlling the operation of a high flow oxygen device (1) according to claim 1, wherein the high flow oxygen device (1) comprises a tracer source provided in close vicinity of the first inlet, releasing a tracer material into the oxygen, and a sensor in close vicinity of the blower (3), detecting the tracer wherein the control unit activates the blower (3) if the sensor detects tracer material such that the pressure reading of the bidirectional flow sensor () becomes equal or greater than zero.
